# EUROPEAN PATENT APPLICATION

(11) **EP 2 194 124 A1**
(43) Date of publication of application: **09.06.2010**
(21) Application number: 10158274.0
(22) Date of filing: 19.09.2006
(51) Int. Cl.: C12N 9/12, A61K 38/45

(54) **Protein kinase c peptide modulators of angiogenesis**

(30) Priority: 19.09.2005 US 718508 P
(62) Divisional of application: 06814988.9
(71) Applicant: Kai Pharmaceuticals, Inc., South San Francisco, CA 94080 (US)
(72) Inventor: Chen, Leon, Belmont, CA 94002 (US); Walter, Sarah, Redwood City, CA 94061 (US); Macelan, Derek, Los Altos, CA 94022 (US)
(74) Representative: Irvine, Jonquil Claire

(57) **Abstract**

The present invention provides peptides for inhibiting various protein kinase C isozymes. The peptide can be directed to any region of the protein kinase C isozyme, and in one embodiment, is directed to the V5 domain. The peptide can be conjugated to a carrier, in a releasable or non-releasable manner. The peptides can be used to inhibit angiogenesis and/or vascular permeability. The peptides can be used to treat subjects having, for example, cancer, diabetic blindness, macular degeneration, rheumatoid arthritis, or psoriasis.

## Description

### Technical Field

The disclosed invention relates to the use of peptide modulators of protein kinase C isoforms to prevent or inhibit angiogenesis and/or prevent or inhibit vascular permeability.

### Background Art

Angiogenesis occurs in the healthy body for healing wounds and for restoring blood flow to tissues after injury or insult. Angiogenesis is also associated with a number of disease states such as cancer, diabetic blindness, wet age-related macular degeneration, rheumatoid arthritis, psoriasis, atheroma, Kaposi's sarcoma, haemangioma, acute and chronic nephropathies, arterial restenosis, autoimmune diseases, acute inflammation, lymphoedema, endometriosis, dysfunctional uterine bleeding and more than 70 other conditions. In these and other disease states, inappropriate development of new blood vessels, also known as undesirable angiogenesis, can serve to feed diseased tissues, destroy normal tissues, and in the case of cancer, the new vessels can facilitate tumor metastases.

### The Angiogenesis Process

The process of angiogenesis typically begins with the production and release of angiogenic growth factors at the site of injury which diffuse into nearby tissues. These growth factors bind to and activate endothelial cells of nearby preexisting blood vessels. Through a complex signal cascade system, the activated endothelial cells begin to proliferate and alter the environment proximal to them. The proliferating endothelial cells migrate out toward the source of the angiogenic growth factors, laying the foundation of new blood vessels. The endothelial cells ultimately form tubes and vessels which serve to supply blood to the areas secreting the angiogenic growth hormones.

Therapies regarding the modulation of angiogenesis are becoming more and more important in medicine. By one estimate, more than $4 billion has been invested in the research and development of angiogenesis-based medicines, making this one of the most heavily funded areas of medical research in human history.

### Protein Kinase C

Protein kinase C (PKC) is a key enzyme in signal transduction involved in a variety of cellular functions, including cell growth, regulation of gene expression and ion channel activity. The PKC family of isozymes includes at least 11 different protein kinases which can be divided into at least three subfamilies based on their homology and sensitivity to activators. Members of the classical or cPKC subfamily, alpha, beta (β_{I}, β_{II}), and gamma isozymes, contain four homologous domains (C1, C2, C3 and C4) inter-spaced with isozyme-unique (variable or V) regions, and require calcium, phosphatidylserine (PS), and diacylglycerol (DG) or phorbol esters for activation. Members of the novel or nPKC subfamily, delta, epsilon, eta, and theta isozymes, lack the C2 homologous domain and do not require calcium for activation. Finally, members of the atypical or aPKC subfamily, zeta and lambda/iota isozymes, lack both the C2 and one half of the C1 homologous domains and are insensitive to DG, phorbol esters and calcium.

Studies on the subcellular distribution of PKC isozymes demonstrate that activation of PKC results in its redistribution in the cells (also termed translocation), such that activated PKC isozymes associate with the plasma membrane, cytoskeletal elements, nuclei, and other subcellular compartments.

It appears that the unique cellular functions of different PKC isozymes are determined by their subcellular location. For example, activated β_{I} PKC is found inside the nucleus, whereas activated β_{II} PKC is found at the perinucleus and cell periphery of cardiac myocytes. Further, in the same cells, epsilon PKC binds to cross-striated structures (possibly the contractile elements) and cell-cell contacts following activation or after addition of exogenous activated epsilon PKC to fixed cells. The localization of different PKC isozymes to different areas of the cell in turn appears due to binding of the activated isozymes to specific anchoring molecules termed Receptors for Activated C-Kinase (RACKs).

RACKs are thought to function by selectively anchoring activated PKC isozymes to their respective subcellular sites. RACKs bind only activated PKC and are not necessarily substrates of the enzyme. Nor is the binding to RACKs mediated via the catalytic domain of the kinase. Translocation of PKC reflects binding of the activated enzyme to RACKs anchored to the cell particulate fraction and the binding to RACKs is required for PKC to produce its cellular responses. Inhibition of PKC binding to RACKs in vivo inhibits PKC translocation and PKC-mediated function.

cDNA clones encoding RACK1 and RACK2 have been identified. Both are homologs of the beta subunit of G proteins, a receptor for another translocating protein kinase, the beta-adrenergic receptor kinase, beta-ARK. Similar to G-proteins, RACK1, and RACK2 have seven WD40 repeats. Recent data suggest that RACK1 is a-selective anchoring protein for activated β_{II} PKC.

Translocation of PKC is required for proper function of PKC isozymes. Peptides that mimic either the PKC-binding site on RACKs or the RACK-binding site on PKC are isozyme-specific translocation inhibitors of PKC that selectively inhibit the function of the enzyme *in vivo.*

### Disclosure of Invention

The current invention contemplates an isolated protein kinase C (PKC) beta or delta inhibitory peptide, said peptide having activity for the inhibition of angiogenesis and/or the inhibition of vascular permeability. In certain embodiments, the peptide comprises an amino acid sequence that has greater than 50% sequence identity with a peptide selected from the group consisting of SEQ ID NOs: 5 conjugated via a disulfide bond to 6, 5 conjugated via a disulfide bond to 7, 5 conjugated via a disulfide bond to 8, 5 conjugated via a disulfide bond to 11, 5 conjugated via a disulfide bond to 12, and SEQ ID NOs:12, 13, 15, 16, 17, 18, 19, 20, 21, 22, and 23. In other embodiments, the peptide has a sequence selected from the group consisting of SEQ ID NOs: 5 conjugated via a disulfide bond to 6, 5 conjugated via a disulfide bond to 7, 5 conjugated via a disulfide bond to 8, 5 conjugated via a disulfide bond to 11, 5 conjugated via a disulfide bond to 12, and SEQ ID NOs:12, 13, 15, 16, 17, 18, 19, 20, 21, 22, and 23. In further embodiments, the peptide is conjugated to a carrier, including, but not limited to poly-Arg, TAT, and Drosophila Antennapedia homeodomain. Peptides conjugated to a carrier include those having the sequence identified as SEQ ID NO: 6, 7, 8, and 11.

The invention also encompasses an isolated linear peptide having greater than 50% sequence identity with a peptide selected from the group consisting of SEQ ID NOs: 13, and 15-23. The peptide can be selected from the group consisting of SEQ ID NOs: 13, and 15-23.

Also encompassed in the invention are isolated PKC beta I V5 peptides comprising an amino acid sequence that has greater than 50% sequence identity with a peptide selected from the group consisting of SEQ ID NOs: 5 conjugated via a disulfide bond to 6, and SEQ ID NOs:13, 18, 20, 21, 22, 23, and having activity as an antagonist of a beta PKC, isolated PKC beta II V5 peptides comprising an amino acid sequence that has greater than 50% sequence identity with a peptide selected from the group consisting of SEQ ID NOs: 5 conjugated via a disulfide bond to 7, and SEQ ID NOs:16, and 19, and isolated PKC delta V5 peptides comprising an amino acid sequence that has greater than 50% sequence identity with a peptide selected from the group consisting of SEQ ID NOs: 5 conjugated via a disulfide bond to 12, and SEQ ID NO:12, and having activity as an antagonist of delta PKC.

Such peptides can be chemically synthesized or recombinantly produced.

Pharmaceutical formulations comprising a pharmaceutically acceptable excipient and the peptides of the invention are also contemplated.

The invention encompasses methods of using the disclosed peptides. In certain embodiments, the peptides are used to inhibit angiogenesis and/or vascular permeability. One method for inhibiting angiogenesis comprises treating an angiogenic endothelial cell with an inhibitory amount of an isolated protein kinase C (PKC) inhibitory peptide, whereby angiogenesis is inhibited. Another method of inhibiting vascular permeability comprises treating an endothelial cell with an inhibitory amount of an isolated protein kinase C (PKC) inhibitory peptide, whereby vascular permeability is inhibited. In further embodiments, the cell is directly contacted with the inhibitory peptide. The PKC inhibitory peptide can inhibit a classical PKC isozyme, beta I or beta II PKC, or a novel PKC isozyme, such as delta PKC. In certain methods, the PKC inhibitory peptide is conjugated to a carrier, and has greater than 50% sequence identity with a peptide selected from the group consisting of CKLFIMN (SEQ ID NO: 6), CQEVIRN (SEQ ID NO: 7), and CSLNPEWNET (SEQ ID NO: 8), or is selected from the group consisting of CKLFIMN (SEQ ID NO: 6), CQEVIRN (SEQ ID NO: 7), and CSLNPEWNET (SEQ ID NO: 8). In other methods, the PKC inhibitory peptide has greater than 50% sequence identity with a peptide selected from the group consisting of SEQ ID NOs:5 conjugated via a disulfide bond to 6, 5 conjugated via a disulfide bond to 7, 5 conjugated via a disulfide bond to 8, 5 conjugated via a disulfide bond to 11, 5 conjugated via a disulfide bond to 12, and SEQ ID NOs:12, 13, 15, 16, 17, 18, 19, 20, 21, 22, and 23, or is selected from the group consisting of SEQ ID NOs: 5 conjugated via a disulfide bond to 6, 5 conjugated via a disulfide bond to 7, 5 conjugated via a disulfide bond to 8, 5 conjugated via a disulfide bond to 11, 5 conjugated via a disulfide bond to 12, and SEQ ID NOs:12, 13, 15, 16, 17, 18, 19, 20, 21, 22, and 23. The PKC inhibitory peptide can be CYSDKNLIDSM (SEQ ID NO: 12). Additionally, the PKC inhibitory peptide can have greater than 50% sequence identity with CSFNSYELGSL (SEQ ID NO: 11) conjugated to a carrier, or can comprise SEQ ID NO: 11 conjugated to a carrier.

Exemplary disorders that can be treated using the peptides include cancer, diabetic blindness, macular degeneration, rheumatoid arthritis, or psoriasis. The peptides can be administered in a variety of routes that are dependent on the disorder to be treated. In one embodiment, the peptide is administered to an ocular tissue of the subject, particularly for the treatment of macular degeneration.

The methods can further comprise treating the cell with an anti-angiogenic agent, which in certain embodiments, inhibits at least one of the group consisting of VEGF, FGF, PDGFB, EGF, LPA, HGF, PD-ECF, IL-8, angiogenin, TNF-alpha, TGF- beta, TGF- alpha, proliferin, and PLGF.

### Brief Description of Drawings

Figure 1 shows an example of the scoring in the corneal angiogenesis assay.

Figures 2A-D show photographs demonstrating that rabbit corneas treated with the β_{II} PKC specific inhibitor substantially prevented VEGF-induced neovascularization when measured at days 7 and 10.

Figures 3A-C show the impact of β_{I} and β_{II} PKC specific inhibitors and an alpha, beta, gamma PKC inhibitor in comparison to a scrambled control peptide and a PKC regulator in the corneal system. The figures show the angiogenesis scores over time (A and B), and on day 12 (C).

Figure 4 shows the impact of delta PKC isozyme specific inhibitors on angiogenesis in comparison to a control peptide or PKC regulator.

Figures 5A-C show the result of two beta PKC inhibitors in the Miles assay, demonstrating that both peptides reduced vascular permeability in comparison to the vehicle alone.

Figures 6 A-D show the results of two beta PKC inhibitors and one classical PKC inhibitor in comparison to a control peptide in the Miles assay.

### Best Mode(s) for Carrying Out the Invention

The disclosed invention relates to the use of peptide modulators of various protein kinase C isozymes to prevent or inhibit angiogenesis, and/or to prevent or inhibit undesired vascular permeability. A peptide modulator of a PKC isozyme is a peptide which either promotes or inhibits the activity of one or more PKC isozymes. In a preferred embodiment, the peptide modulator acts specifically on a single PKC isozyme. Non-specific PKC modulating peptides are also contemplated.

The current invention specifically contemplates an isolated protein kinase C (PKC) beta or delta inhibitory peptide, said peptide having activity for the inhibition of angiogenesis and/or the inhibition of vascular permeability. In certain embodiments, the peptide comprises an amino acid sequence that has greater than 50% sequence identity with a peptide selected from the group consisting of SEQ ID NOs: 5 conjugated via a disulfide bond to 6, 5 conjugated via a disulfide bond to 7, 5 conjugated via a disulfide bond to 8, 5 conjugated via a disulfide bond to 11, 5 conjugated via a disulfide bond to 12, and SEQ ID NOs:12, 13, 15, 16, 17, 18, 19, 20, 21, 22, and 23. In other embodiments, the peptide has a sequence selected from the group consisting of SEQ ID NOs: 5 conjugated via a disulfide bond to 6, 5 conjugated via a disulfide bond to 7, 5 conjugated via a disulfide bond to 8, 5 conjugated via a disulfide bond to 11, 5 conjugated via a disulfide bond to 12, and SEQ ID NOs:12, 13, 15, 16, 17, 18, 19, 20, 21, 22, and 23. In further embodiments, the peptide is conjugated to a carrier, including, but not limited to poly-Arg, TAT, and Drosophila Antennapedia homeodomain. The invention specifically contemplates the conjugation of peptides having the sequence identified as SEQ ID NO: 6, 7, 8, and 11 to a carrier.

The invention also encompasses an isolated linear peptide having greater than 50% sequence identity with a peptide selected from the group consisting of SEQ ID NOs: 13, and 15-23. The peptide can be selected from the group consisting of SEQ ID NOs: 13, and 15-23.

Also encompassed in the invention are isolated PKC beta I V5 peptides comprising an amino acid sequence that has greater than 50% sequence identity with a peptide selected from the group consisting of SEQ ID NOs: 5 conjugated via a disulfide bond to 6, and SEQ ID NOs:13, 18, 20, 21, 22,and 23, and having activity as an antagonist of a beta I PKC, isolated PKC beta II V5 peptides comprising an amino acid sequence that has greater than 50% sequence identity with a peptide selected from the group consisting of SEQ ID NOs: 5 conjugated via a disulfide bond to 7, and SEQ ID NOs:16, and 19, and having activity as an antagonist of a beta II PKC, and isolated PKC delta V5 peptides comprising an amino acid sequence that has greater than 50% sequence identity with a peptide selected from the group consisting of SEQ ID NOs: 5 conjugated via a disulfide bond to 12, and SEQ ID NO:12, and having activity as an antagonist of delta PKC.

Such peptides can be chemically synthesized or recombinantly produced.

Pharmaceutical formulations comprising a pharmaceutically acceptable excipient and the peptides of the invention are also contemplated.

The invention described herein contemplates the administration of one or more PKC activity modulating peptides to a subject in order to inhibit undesirable angiogenesis activity and/or to prevent or inhibit undesired vascular permeability. Cancers involving tumors, diabetes-related neovascularization, wet age-related macular degeneration, rheumatoid arthritis, psoriasis, atheroma, Kaposi's sarcoma, haemangioma, acute and chronic nephropathies, arterial restenosis, autoimmune diseases, acute inflammation, lymphoedema, endometriosis, and dysfunctional uterine bleeding are just a few examples of disease states characterized as resulting in undesirable angiogenic activity and/or undesirable vascular permeability.

The invention encompasses methods of using the disclosed peptides. In certain embodiments, the peptides are used to inhibit angiogenesis and/or vascular permeability. One method for inhibiting angiogenesis comprises treating an angiogenic endothelial cell with an inhibitory amount of an isolated protein kinase C (PKC) inhibitory peptide, whereby angiogenesis is inhibited. Another method of inhibiting vascular permeability comprises treating an endothelial cell with an inhibitory amount of an isolated protein kinase C (PKC) inhibitory peptide, whereby vascular permeability is inhibited. In further embodiments, the cell is directly contacted with the inhibitory peptide. The PKC inhibitory peptide can inhibit a classical PKC isozyme, beta I or beta II PKC, or a novel PKC isozyme, such as delta PKC. In certain methods, the PKC inhibitory peptide is conjugated to a carrier, and has greater than 50% sequence identity with a peptide selected from the group consisting of CKLFIMN (SEQ ID NO: 6), CQEVIRN (SEQ ID NO: 7), and CSLNPEWNET (SEQ ID NO: 8), or is selected from the group consisting of CKLFIMN (SEQ ID NO: 6), CQEVIRN (SEQ ID NO: 7), and CSLNPEWNET (SEQ ID NO: 8). In other methods, the PKC inhibitory peptide has greater than 50% sequence identity with a peptide selected from the group consisting of SEQ ID NOs:5 conjugated via a disulfide bond to 6, 5 conjugated via a disulfide bond to 7, 5 conjugated via a disulfide bond to 8, 5 conjugated via a disulfide bond to 11, 5 conjugated via a disulfide bond to 12, and SEQ ID NOs:12, 13, 15, 16, 17, 18, 19, 20, 21, 22, and 23, or is selected from the group consisting of SEQ ID NOs: 5 conjugated via a disulfide bond to 6, 5 conjugated via a disulfide bond to 7, 5 conjugated via a disulfide bond to 8, 5 conjugated via a disulfide bond to 11, 5 conjugated via a disulfide bond to 12, and SEQ ID NOs: 12, 13, 15, 16, 17, 18, 19, 20, 21, 22, and 23. The PKC inhibitory peptide can be CYSDKNLIDSM (SEQ ID NO: 12). Additionally, the PKC inhibitory peptide can have greater than 50% sequence identity with CSFNSYELGSL (SEQ ID NO: 11) conjugated to a carrier, or can comprise SEQ ID NO: 11 conjugated to a carrier.

Non-limiting and exemplary disorders that can be treated using the peptides include tumors, diabetes-related neovascularization, wet age-related macular degeneration, rheumatoid arthritis, psoriasis, atheroma, Kaposi's sarcoma, haemangioma, acute and chronic nephropathies, arterial restenosis, autoimmune diseases, acute inflammation, lymphoedema, endometriosis, and dysfunctional uterine bleeding. The peptides can be administered in any number of routes that are dependent on the disorder to be treated, and are described further herein. In one embodiment, the peptide is administered to an ocular tissue of the subject, particularly for the treatment of macular degeneration.

The methods can further comprise treating the cell with an anti-angiogenic agent, which in certain embodiments, inhibits at least one of the group consisting of VEGF, FGF, PDGFB, EGF, LPA, HGF, PD-ECF, IL-8, angiogenin, TNF-alpha, TGF- beta, TGF- alpha, proliferin, and PLGF.

Within this application, unless otherwise stated, definitions of the terms and illustration of the techniques of this application may be found in any of several well-known references such as: Sambrook, J., et al., Molecular Cloning: A Laboratory Manual, Cold Spring Harbor Laboratory Press (1989); Goeddel, D., ed., Gene Expression Technology, Methods in Enzymology, 185, Academic Press, San Diego, CA (1991); "Guide to Protein Purification" in Deutshcer, M.P., ed., Methods in Enzymology, Academic Press, San Diego, CA (1989); Innis, et al., PCR Protocols: A Guide to Methods and Applications, Academic Press, San Diego, CA (1990); Freshney, R.I., Culture of Animal Cells: A Manual of Basic Technique, 2nd Ed., Alan Liss, Inc. New York, NY (1987); Murray, E.J., ed., Gene Transfer and Expression Protocols, pp. 109-128, The Humana Press Inc., Clifton, NJ and Lewin, B., Genes VI, Oxford University Press, New York (1997).

The present invention may be understood more readily by reference to the following detailed description of the preferred embodiments of the invention and the Examples included herein. However, before the present methods are disclosed and described, it is to be understood that this invention is not limited to specific nucleic acids, specific polypeptides, specific cell types, specific host cells, specific conditions, or specific methods, etc., as such may, of course, vary, and the numerous modifications and variations therein will be apparent to those skilled in the art. It is also to be understood that the terminology used herein is for the purpose of describing specific embodiments only and is not intended to be limiting. It is further to be understood that unless specifically defined herein, the terminology used herein is to be given its traditional meaning as known in the relative art.

### Role of PKC Isozymes in Angiogenesis

PKC activity plays a role in angiogenesis. Endothelial cells responding to hypoxic conditions modulate the activity of PKC isozymes. These effects have been described in the literature, for example in cardiac tissue. Using the diabetic retinopathy as a model illustrates the role of PKC isozymes in angiogenesis.

Conditions of elevated high blood glucose levels (diabetes) results in the production of diacylglycerol and advanced glycation end products (AGEs). AGEs are a heterogeneous group of molecules formed from the nonenzymatic reaction of reducing sugars with free amino groups of proteins, lipids, and nucleic acids. Certain AGEs can bind to cell-surface AGE-binding receptors, possibly leading to cellular activation and the generation of VEGF, a key component to angiogenesis. These conditions also lead to the formation of free radicals and the activation of various isozymes of PKC, such as the alpha, beta, delta and epsilon isozymes in the retina. Increased PKC activity is linked to expression of ET-1, which is a vasoconstrictor. Inhibition of PKC activity, for example, is thought to reduce VEGF production and thus reduce or inhibit angiogenesis and neovascularization.

Apoptosis of pericytes, thickening of retinal vessel basement membranes and hyperpermeability also play a role in diabetic retinopathy and point to a role for PKC in the development of unhealthy neovascularization. Pericytes are important for protection of endothelial cells against lipid-peroxide-induced injury. PKC activity can increase production of TGF-□, ECM proteins (fibrinogen, type IV collagen) leading to basement membrane thickening. It has also been shown that activation of □PKC increases vascular permeability. Furthermore, delta PKC has been linked with VEGF secretion under high glucose conditions in RPE cells.

Evidence that interfering with PKC function impacts diabetic retinopathy includes work done in transgenic animals and in-rat models. For example, transgenic mice that overexpress □PKC have "diabetes-like" symptoms - increased neovascularization in response to ischemia. Mouse knockouts of □PKC have reduced neovascularization in response to ischemia. Diabetic rats treated with LY333531 (□PKC-selective inhibitor from Eli Lilly) have improved retinal blood flow compared to untreated diabetic rats. And it has been shown that □PKC is upregulated and activated (translocated) in diabetic models. Animals injected with VEGF can increase retinal vascular permeability - pretreatment with LY333531 has been shown to prevent this result. Also, using the corneal angiogenesis model in rats, LY317615 (also a beta-PKC selective inhibitor from Eli Lilly) given systemically was able to prevent new vessel growth.

### Wet Age-Related Macular Degeneration (AMD) and the role of PKC

PKC activity has been postulated as playing a role in the development of wet age-related macular degeneration (wet AMD). In this disease state, damage to the retinal pigment epithelium (RPE) is thought to cause secretion of angiogenic growth factors, cytokines and proteases which promote or induce an angiogenic response. Clinical samples of membranes with choroidal neovascularization (CNV) have shown elevated levels of vascular endothelial growth factor (VEGF), platelet derived growth factor (PDGF), transforming growth factor (TGF), all of these factors are known to facilitate angiogenesis or neovascularization.

Neovascular structures, composed of cells such as RPEs, vascular endothelial cells, fibroblasts and macrophages intrude into the intraocular space. These structures can destabilize the retina, causing damage and a decrease in visual acuity. Perhaps more importantly, neovascular structures tend to be poorly formed and generally fragile. Thus, these neovascular structures tend to hemorrhage into the intraocular space, rendering the normally clear vitreous humor opaque.

### Peptide Modulators of PKC Isozymes

A variety of peptide modulators of PKC isozymes have been previously described. For example, U.S. Patent No. 5,783,405 describes a number of peptides which modulate the activity of PKC isozymes, including the beta, theta, delta, epsilon, and gamma isozymes. Pending U.S. Patent Application No. 10/843,271 describes delta PKC modulating peptides and derivatives thereof. U.S. Patent No. 6,165,977 describes epsilon PKC modulation peptides and derivatives thereof. U.S. Patent No. 6,855,693 describes a variety of modulating peptides and modified fragments from the α, β_{I}, β_{II}, γ, δ ε, η, µ, Θ, and ζ isozymes. Each patent and patent application is hereby incorporated by reference in their entirety.

The peptides discussed above can be used alone or in combination with one another. For example, the administration of peptide inhibitors which act on the β_{I} and β_{II;} PKC isozymes in combination with another inhibitor specific for δ PKC is specifically contemplated.

In certain embodiments of the present invention, the compound can be administered with a therapeutically effective amount of an anti-cancer agent, wherein the anti-cancer agent is selected from the group consisting of a chemotherapeutic agent, a radiotherapeutic agent, an anti-angiogenic agent, and an apoptosis-inducing agent. As used herein, a "therapeutically effective amount" is an amount which has a negative effect on angiogenesis or tumor growth. As also used herein, an "anti-cancer agent" refers to a molecule which has a negative effect on angiogenesis, metastasis, or tumor growth. In one embodiment, the anti-cancer agent is an anti- angiogenic agent that inhibits the expression or activity of an angiogenic factor selected from the group consisting of VEGFs, FGFs, PDGFB, EGF, LPA, HGF, PD-ECF, IL-8, angiogenin, TNF-alpha, TGF- beta, TGF- alpha, proliferin, and PLGF. In another embodiment, the anti-cancer agent is an anti-angiogenic agent selected from the group consisting of an agent that inhibits the expression or activity of a matrix metalloproteinase; an agent that interacts with a cell adhesion molecule; and an agent that inhibits the activity of a urokinase; and an agent that inhibits angiogenesis through another mechanism. In particular embodiments, the compounds of the disclosed invention can be used in conjunction with other anti-angiogenesis treatments such as MACUGEN, LUCENTIS, RETAANE, EVIZON, AVASTIN and ARXXANT.

As used herein, "peptide" and "polypeptide" are used interchangeably and refer to a compound made up of a chain of amino acid residues linked by peptide bonds. Unless otherwise indicated, the sequence for peptides is given in the order from the amino terminus to the carboxyl terminus. In particular embodiments, the peptide inhibitor comprises two or more peptides linked by intermolecular disulfide bonds, where the peptides are the same or different. In other embodiments, the peptide inhibitor is 3-25, 6-20, or 6-15, or 6-12 amino acids in length. In certain embodiments, one or more amino acids in the peptide are d-amino acids.

In one embodiment, the inhibitory peptide inhibits binding of the RACK to the PKC V5 domain is inhibited, although inhibition of RACK interaction with other PKC domains, such as the PKC C2 domain for example, is also contemplated. The beta I PKC V5 domain has the sequence KPKARDKRDTSNFDKEFTRQPVELTPTDKLFIMNLDQNEFAGFSYTNPEFVIN V (SEQ ID NO:1); the beta II PKC V5 domain has the sequence KPKACG-RNAENFDRFFTRHPPVLTPPDQEVIRNIDQSEFEGFSFVNSEFLKPEVKS (SEQ ID NO:2); and the delta PKC V5 domain has the sequence RPKVKSPRDYSNFDQEFLNEKARLSYSDKNLIDSMDQSAFAGFSFVNPKFEHL LED (SEQ ID NO:3). In certain embodiments, the inhibitory peptide comprises 3-25, 6-20, 6-15, or 6-12 consecutive residues of SEQ ID NOs:1, 2 or 3, or has greater than 50% sequence identity with a peptide comprising 3-25, 6-20, 6-15, or 6-12 consecutive residues of SEQ ID NOs:1, 2 or 3. Inhibitory peptides that are substantially complementary to a variable domain can also overlap a conserved domain.

To determine the percent homology of two amino acid sequences, the sequences are aligned for optimal comparison purposes (e.g., gaps can be introduced in the sequence of one polypeptide for optimal alignment with the other polypeptide). The amino acid residues at corresponding amino acid positions are then compared. When a position in one sequence is occupied by the same amino acid residue as the corresponding position in the other sequence, then the molecules are identical at that position. As used herein amino acid or nucleic acid "homology" is equivalent to amino acid or nucleic acid "identity". Accordingly, the percent sequence identity between the two sequences is a function of the number of identical positions shared by the sequences (i.e., percent sequence identity = numbers of identical positions/total numbers of positions x 100). Preferably, the isolated amino acid variants included in the present invention are at least about 50-60%, preferably at least about 60-70%, and more preferably at least about 70-75%, 75-80%, 80-85%, 85-90%, or 90-95%, and most preferably at least about 96%, 97%, 98%, 99%, or more identical to an entire amino acid sequence shown in any of SEQ ID NOs: 5 conjugated via a disulfide bond to 6, 5 conjugated via a disulfide bond to 7, 5 conjugated via a disulfide bond to 8, 5 conjugated via a disulfide bond to 11, 5 conjugated via a disulfide bond to 12, and SEQ ID NOs:12, 13, 15, 16, 17, 18, 19, 20, 21, 22, and 23, or to a peptide comprising 3-25, 6-20, 6-15, or 6-12 consecutive residues of SEQ ID NOs: 1, 2 or 3. In another embodiment, the isolated amino acid variants included in the present invention are at least about 50-60%, preferably at least about 60-70%, and more preferably at least about 70-75%, 75-80%, 80-85%, 85-90%, or 90-95%, and most preferably at least about 96%, 97%, 98%, 99%, or more identical to an entire amino acid sequence shown in any of SEQ ID NOs: 6, 7, 8, or 11, where the peptide is conjugated to a carrier.

For the purposes of the invention, the percent sequence identity between two polypeptide sequences is determined using the Vector NTI 6.0 (PC) software package (InforMax, 7600 Wisconsin Ave., Bethesda, MD 20814). A gap opening penalty of 10 and a gap extension penalty of 0.1 are used for determining the percent identity of two polypeptides. All other parameters are set at the default settings.

A peptide or peptide fragment is "derived from" a parent peptide or polypeptide if it has an amino acid sequence that is identical or homologous to at least a contiguous sequence of five amino acid residues of the parent peptide or polypeptide.

A peptide has "isozyme-specific activity" when it acts on a particular PKC isozyme involved in the angiogenesis pathway, as opposed to non-specific peptides or compounds that fail to discriminate between PKC isozymes.

As used herein, "conservative amino acid substitutions" are substitutions which do not result in a significant change in the activity or tertiary structure of a selected polypeptide or protein. Such substitutions typically involve replacing a selected amino acid residue with a different residue having similar physico-chemical properties. Groupings of amino acids by physico-chemical properties are known to those of skill in the art. For example, families of amino acid residues having similar side chains have been defined in the art, and include basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine).

Another aspect of the invention pertains to the use of isolated PKC polypeptides, and biologically active portions thereof, and in one embodiment, pertains to the use of PKC V5 domain polypeptides. An "isolated" or "purified" polypeptide or biologically active portion thereof is free of some of the cellular material when produced by recombinant DNA techniques, or chemical precursors or other chemicals when chemically synthesized. The language "substantially free of cellular material" includes preparations of PKC domain polypeptides in which the polypeptide is separated from some of the cellular components of the cells in which it is naturally or recombinantly produced. In one embodiment, the language "substantially free of cellular material" includes preparations of a PKC domain polypeptide having less than about 30% (by dry weight) of non-PKC material (also referred to herein as a "contaminating polypeptide"), more preferably less than about 20% of non-PKC material, still more preferably less than about 10% of non-PKC material, and most preferably less than about 5% non-PKC material.

When the PKC polypeptide or biologically active portion thereof is recombinantly produced, it is also preferably substantially free of culture medium, i.e., culture medium represents less than about 20%, more preferably less than about 10%, and most preferably less than about 5% of the volume of the polypeptide preparation. The language "substantially free of chemical precursors or other chemicals" includes preparations of PKC domain polypeptides in which the polypeptide is separated from chemical precursors or other chemicals that are involved in the synthesis of the polypeptide. In one embodiment, the language "substantially free of chemical precursors or other chemicals" includes preparations of a PKC domain polypeptide having less than about 30% (by dry weight) of chemical precursors or other chemicals, more preferably less than about 20% chemical precursors or other chemicals, still more preferably less than about 10% chemical precursors or other chemicals, and most preferably less than about 5% chemical precursors or other chemicals. In preferred embodiments, isolated polypeptides, or biologically active portions thereof, lack contaminating polypeptides from the same organism from which the PKC domain polypeptide is derived.

The PKC polypeptides can be conjugated to a carrier. Non-limiting methods for conjugating the peptide to the carrier include conjugation via a disulfide bond, and synthesis as a single chain or linear polypeptide. The carrier can be conjugated to the PKC polypeptide via a linker. In one embodiment, the linker is a 1-5 amino acid peptide, a 2-4 amino acid peptide, or a 2-3 amino acid peptide.

The carrier is any compound that allows cell penetration. Non-limiting examples of carriers include poly-Arg, TAT, and the Drosophila Antennapedia homeodomain. The sequence of TAT is YGRKKRRQRRR (SEQ ID NO:4). The TAT sequence can also have an N-teminal cysteine for use in conjugation to the peptide, CYGRKKRRQRRR (SEQ ID NO:5). In certain embodiments, the carrier comprises 3-25 residues, 4-20 residue, 5-15 residues or 6-12 residues in length. The selection of a carrier is well known to those of skill in the art.

### Formulations

Methods of preparing these formulations or compositions include the step of bringing into association a compound of the present invention with the carrier and, optionally, one or more accessory ingredients. In general, the formulations are prepared by uniformly and intimately bringing into association a compound of the present invention with liquid carriers, or finely divided solid carriers, or both, and then, if necessary, shaping the product.

Pharmaceutical compositions of the present invention suitable for parenteral administration comprise one or more compounds of the invention in combination with one or more pharmaceutically-acceptable sterile isotonic aqueous or nonaqueous solutions, dispersions, suspensions or emulsions, or sterile powders which may be reconstituted into sterile injectable solutions or dispersions just prior to use, which may contain sugars, alcohols, antioxidants, buffers, bacteriostats, solutes which render the formulation isotonic with the blood of the intended recipient or suspending or thickening agents.

Examples of suitable aqueous and nonaqueous carriers which may be employed in the pharmaceutical compositions of the invention include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

These compositions may also contain adjuvants such as preservatives, wetting agents, emulsifying agents and dispersing agents. Prevention of the action of microorganisms upon the compounds of the present invention may be ensured by the inclusion of various antibacterial and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption such as aluminum monostearate and gelatin.

In some cases, in order to prolong the effect of a drug, it is desirable to slow the absorption of the drug from subcutaneous or intramuscular injection. This may be accomplished by the use of a liquid suspension of crystalline or amorphous material having poor water solubility. The rate of absorption of the drug then depends upon its rate of dissolution which, in turn, may depend upon crystal size and crystalline form. Alternatively, delayed absorption of a parenterally-administered drug form is accomplished by dissolving or suspending the drug in an oil vehicle.

Injectable depot forms are made by forming microencapsule matrices of the compounds of the present invention in biodegradable polymers such as polylactide-polyglycolide. Depending on the ratio of drug to polymer, and the nature of the particular polymer employed, the rate of drug release can be controlled. Examples of other biodegradable polymers include poly(orthoesters) and poly(anhydrides). Depot injectable formulations are also prepared by entrapping the drug in liposomes or microemulsions which are compatible with body tissue.

Formulations of the invention suitable for oral administration may be in the form of capsules, cachets, pills, tablets, lozenges (using a flavored basis, usually sucrose and acacia or tragacanth), powders, granules, or as a solution or a suspension in an aqueous or non-aqueous liquid, or as an oil-in-water or water-in-oil liquid emulsion, or as an elixir or syrup, or as pastilles (using an inert base, such as gelatin and glycerin, or sucrose and acacia) and/or as mouth washes and the like, each containing a predetermined amount of a compound of the present invention as an active ingredient. A compound of the present invention may also be administered as a bolus, electuary or paste.

When the compounds of the present invention are administered as pharmaceuticals, to humans and animals, they can be given per se or as a pharmaceutical composition containing, for example, 0.1 to 99% (more preferably, 10 to 30%) of active ingredient in combination with a pharmaceutically acceptable carrier.

These compounds may be administered to humans and other animals for therapy by any suitable route of administration, including orally, nasally, as by, for example, a spray, rectally, intravaginally, parenterally, intravenously, subcutaneously, intracisternally and topically, as by powders, ointments or drops, including buccally and sublingually.

Regardless of the route of administration selected, the compounds of the present invention, which may be used in a suitable hydrated form, and/or the pharmaceutical compositions of the present invention, are formulated into pharmaceutically-acceptable dosage forms by conventional methods known to those of skill in the art.

Actual dosage levels of the active ingredients in the pharmaceutical compositions of the present invention may be varied so as to obtain an amount of the active ingredient which is effective to achieve the desired therapeutic response for a particular patient, composition, and mode of administration, without being toxic to the patient.

The selected dosage level will depend upon a variety of factors including the activity of the particular compound of the present invention employed, or the ester, salt or amide thereof, the route of administration, the time of administration, the rate of excretion or metabolism of the particular compound being employed, the rate and extent of absorption, the duration of the treatment, other drugs, compounds and/or materials used in combination with the particular compound employed, the age, sex, weight, condition, general health and prior medical history of the patient being treated, and like factors well known in the medical arts.

A physician or veterinarian having ordinary skill in the art can readily determine and prescribe the effective amount of the pharmaceutical composition required. For example, the physician or veterinarian could start doses of the compounds of the invention employed in the pharmaceutical composition at levels lower than that required in order to achieve the desired therapeutic effect and gradually increase the dosage until the desired effect is achieved.

In general, a suitable daily dose of a compound of the invention will be that amount of the compound which is the lowest dose effective to produce a therapeutic effect. Such an effective dose will generally depend upon the factors described above. Generally, oral, intravenous, intracerebroventricular and subcutaneous doses of the compounds of the present invention for a patient, when used for the indicated analgesic effects, will range from about 0.0001 to about 100 mg per kilogram of body weight per day.

If desired, the effective daily dose of the active compound may be administered as two, three, four, five, six or more sub-doses administered separately at appropriate intervals throughout the day, optionally, in unit dosage forms. Preferred dosing is one administration per day.

While it is possible for a compound of the present invention to be administered alone, it is preferable to administer the compound as a pharmaceutical formulation (composition).

The subject receiving this treatment is any animal in need, including primates, in particular humans, and other mammals such as equines, cattle, swine and sheep; and poultry and pets in general.

The compound of the invention can be administered as such or in admixtures with pharmaceutically acceptable carriers and can also be administered in conjunction with antimicrobial agents such as penicillins, cephalosporins, aminoglycosides and glycopeptides. Conjunctive therapy thus includes sequential, simultaneous and separate administration of the active compound in a way that the therapeutical effects of the first administered one is not entirely disappeared when the subsequent is administered.

### Possible Routes of Administration for Disclosed Compounds

These compounds may be administered to humans and other animals for therapy by any suitable route of administration. As used herein, the term "route" of administration is intended to include, but is not limited to subcutaneous injection, intravenous injection, intraocular injection, intradermal injection, intramuscular injection, intraperitoneal injection, intratracheal administration, epidural administration, inhalation, intranasal administration, oral administration, sublingual administration, buccal administration, rectal administration, vaginal administration, intracisternal administration and topical administration. The disclosed compounds have efficacy when administered systemically.

As described above, the compound can be administered with a therapeutically effective amount of an anti-cancer agent, wherein the anti-cancer agent is selected from the group consisting of a chemotherapeutic agent, a radiotherapeutic agent, an anti-angiogenic agent, and an apoptosis-inducing agent. The particular combination of therapies (therapeutics or procedures) to employ in a combination regimen will take into account compatibility of the desired therapeutics and/or procedures and the desired therapeutic effect to be achieved. It will also be appreciated that the therapies employed may achieve a desired effect for the same disorder (for example, an inventive compound may be administered concurrently with another agent used to treat the same disorder), or they may achieve different effects (e.g., control of any adverse effects). As used herein, additional therapeutic agents that are normally administered to treat or prevent a particular disease, or condition, are known as "appropriate for the disease, or condition, being treated".

A combination treatment of the present invention as defined herein may be achieved by way of the simultaneous, sequential or separate administration of the individual components of said treatment.

The compounds of this invention or pharmaceutically acceptable compositions thereof may also be incorporated into compositions for coating implantable medical devices, such as prostheses, artificial valves, vascular grafts, stents and catheters. Accordingly, the present invention, in another aspect, includes a composition for coating an implantable device comprising a compound of the present invention as described generally above, and a carrier suitable for coating said implantable device. In still another aspect, the present invention includes an implantable device coated with a composition comprising a compound of the present invention as described generally above, and a carrier suitable for coating said implantable device.

Vascular stents, for example, have been used to overcome restenosis (re-narrowing of the vessel wall after injury). However, patients using stents or other implantable devices risk clot formation or platelet activation. These unwanted effects may be prevented or mitigated by pre-coating the device with a pharmaceutically acceptable composition comprising a kinase inhibitor. Suitable coatings and the general preparation of coated implantable devices are described in U.S. Pat. Nos. 6,099,562; 5,886,026; and 5,304,121, herein incorporated by reference in their entirety. The coatings are typically biocompatible polymeric materials such as a hydrogel polymer, polymethyldisiloxane, polycaprolactone, polyethylene glycol, polylactic acid, ethylene vinyl acetate, and mixtures thereof The coatings may optionally be further covered by a suitable topcoat of fluorosilicone, polysaccarides, polyethylene glycol, phospholipids or combinations thereof to impart controlled release characteristics in the composition.

### Administration for Treating Ocular Diseases

Ophthalmic formulations, and eye drops are contemplated as being within the scope of this invention. In particular embodiments, intravitreal injection and periocular administration are acceptable routes of administration. Single or repeated doses or sustained release administration are also contemplated.

For the treatment of wet macular degeneration, the compounds of the invention can be administered in combination with anti-angiogenic agents, or in combination with medical or surgical procedures, such as photocoagulation, photodynamic therapy, and macular translocation surgery.

### Administration for Treating Cancer

Anti-cancer effects of a method of treatment of the present invention include, but are not limited to, anti-tumour effects, the response rate, the time to disease progression and the survival rate. Anti-tumour effects of a method of treatment of the present invention include but are not limited to, inhibition of tumour growth, tumour growth delay, regression of tumour, shrinkage of tumour, increased time to regrowth of tumour on cessation of treatment, slowing of disease progression. It is expected that when a method of treatment of the present invention is administered to a subject in need of treatment for cancer, said method of treatment will produce an effect, as measured by, for example, one or more of: the extent of the anti-tumour effect, the response rate, the time to disease progression and the survival rate. Anti-cancer effects include prophylactic treatment as well as treatment of existing disease.

With respect to combination therapies, for example, chemotherapeutic agents, other anti-proliferative agents, or surgical or medical techniques may be combined with the compounds of this invention to treat proliferative diseases and cancer. For example, other therapies or anticancer agents that may be used in combination with the inventive anticancer agents of the present invention include surgery, radiotherapy (in but a few examples, gamma.-radiation, neutron beam radiotherapy, electron beam radiotherapy, proton therapy, brachytherapy, and systemic radioactive isotopes, to name a few), endocrine therapy, biologic response modifiers (interferons, interleukins, and tumor necrosis factor (TNF) to name a few), hyperthermia and cryotherapy, agents to attenuate any adverse effects (e.g., antiemetics), and other approved chemotherapeutic drugs, including, but not limited to, alkylating drugs (mechlorethamine, chlorambucil, Cyclophosphamide, Melphalan, Ifosfamide), antimetabolites (Methotrexate), purine antagonists and pyrimidine antagonists (6-Mercaptopurine, 5-Fluorouracil, Cytarabile, Gemcitabine), spindle poisons (Vinblastine, Vincristine, Vinorelbine, Paclitaxel), podophyllotoxins (Etoposide, Irinotecan, Topotecan), antibiotics (Doxorubicin, Bleomycin, Mitomycin), nitrosoureas (Carmustine, Lomustine), inorganic ions (Cisplatin, Carboplatin), enzymes (Asparaginase), and hormones (Tamoxifen, Leuprolide, Flutamide, and Megestrol), Gleevec.TM., adriamycin, dexamethasone, and cyclophosphamide. For a more comprehensive discussion of updated cancer therapies see, e.g., The Merck Manual, Seventeenth Ed. 1999, the entire contents of which are hereby incorporated by reference.

For treating tumors, the compounds of the current invention can be administered, for example, orally, systemically, endoscopically, intratracheally, intralesionally, percutaneously, intravenously, subcutaneously, intraperitoneally or intratumourally.

A biologically effective molecule may be operably linked to the peptide of the invention with a covalent bond or a non-covalent interaction. In specific embodiments, the operably linked biologically effective molecules can alter the pharmacokinetics of the peptides of the above described embodiments of the invention by virtue of conferring properties to the peptide as part of a linked molecule. Some of the properties that the biologically effective molecules can confer on the peptides include, but are not limited to: delivery of a peptide to a discrete location within the body; concentrating the activity of a peptide at a desired location in the body and reducing its effects elsewhere; reducing side effects of treatment with a peptide; changing the permeability of a peptide; changing the bioavailability or the rate of delivery to the body of a peptide; changing the length of the effect of treatment with a peptide; altering the stability of the peptide; altering the rate of the onset and the decay of the effects of a peptide; providing a permissive action by allowing a peptide to have an effect.

### Screening using the Disclosed Compounds

The invention also provides screening methods using the disclosed PKC inhibitory peptides.

In one embodiment, the method comprises using the disclosed PKC inhibitory peptides to identify compounds that modulate angiogenesis and/or vascular permeability. The method alternatively is used to identify compounds that modulate the inhibition of PKC. The method can comprise measuring the activity of a PKC inhibitory peptide as disclosed herein in the presence and absence of a test compound; and selecting the test compound as being effective to modulate angiogenesis and/or vascular permeability if the activity of the peptide is altered in the presence of the test compound in comparison to activity in the presence of a control peptide. The measuring step can involve measuring the activity of said peptide in a competitive binding assay in the presence of the test compound. The selecting step can involve selecting the test compound as being effective if binding of the peptide is decreased in the presence of the test compound. The methods can occur within a cell, or in a cell-free environment. In certain embodiments, the test compound is an organic compound.

The invention further contemplates a process for making a compound that modulates the inhibition of one or more PKC isozymes by a PKC inhibitory peptide, comprising: carrying out a method as described herein to identify a compound that modulates the inhibition of one or more PKC isozymes by a PKC inhibitory peptide; and manufacturing the compound.

### Kits Comprising the Disclosed Compounds

The invention also provides kits for carrying out the therapeutic regimens of the invention. Such kits comprise therapeutically effective amounts of a peptide having isozyme-specific inhibitory activity for PKC beta and/or delta, in pharmaceutically acceptable form, alone or in combination with other agents, in pharmaceutically acceptable form. Preferred pharmaceutical forms include peptides in combination with sterile saline, dextrose solution, buffered solution, or other pharmaceutically acceptable sterile fluid. Alternatively, the composition may be lyophilized or desiccated. In this instance, the kit may further comprise a pharmaceutically acceptable solution, preferably sterile, to form a solution for injection purposes. In another embodiment, the kit may further comprise a needle or syringe, preferably packaged in sterile form, for injecting the composition. In other embodiments, the kit further comprises an instruction means for administering the composition to a subject. The instruction means can be a written insert, an audiotape, an audiovisual tape, or any other means of instructing the administration of the composition to a subject.

In one embodiment, the kit comprises (i) a first container containing a peptide having isozyme-specific inhibitory activity for PKC beta; (ii) a second container containing a peptide having isozyme-specific inhibitory activity for PKC delta; and (iii) instruction means for use.

In another embodiment, the kit comprises (i) a first container containing a peptide having isozyme-specific inhibitory activity for a PKC beta or delta domain; (ii) a second container containing an anti-angiogenic agent; and (iii) instruction means for use.

In one embodiment the peptide has isozyme-specific inhibitory activity for a PKC beta or delta V5 domain. In another embodiment, the peptide has isozyme-specific inhibitory activity for a non-V5 domain.

In one embodiment, the anti-angiogenic agent inhibits at least one of the group consisting of VEGF, FGF, PDGFB, EGF, LPA, HGF, PD-ECF, IL-8, angiogenin, TNF-alpha, TGF- beta, TGF- alpha, proliferin, and PLGF.

In related aspects, the invention provides articles of manufacture that comprise the contents of the kits described above. For instance, the invention provides an article of manufacture comprising an effective amount of a peptide having isozyme-specific inhibitory activity for PKC beta and/or delta, alone or in combination with other agents, and instructions indicating use for treating diseases described herein.

The following examples are offered to illustrate but not to limit the invention.

### Example 1

### Inhibition of PKC Affects VEGF Production

PKC has been also been implicated in the regulation of VEGF expression in vascular smooth muscle cells and retinal endothelial cells. (*See,* Soucy et al., Chem. Res. Toxicol. 17:555-63 and Mamputu et al., J. Diabetes Complications 16:284-93 (2002), respectively.) Experiments conducted in primary rat retinal pigment epithelial cells (RPEs) demonstrated that following exposure to PMA for 24 hours, PKC-delta was significantly down regulated in the RPEs. Secretion of VEGF in normal or high glucose, or hypoxia was significantly reduced following treatment with PMA for 24 hours but not with a PKC-zeta specific inhibitor. These experiments showed that, in conditions of high glucose and hypoxia, PKC isozymes are activated and are necessary for VEGF expression. Secretion of VEGF is enhanced in hypoxia and appears to be regulated by PKC-delta. RPE cells may contribute to the pathogenesis of retinopathy caused by high glucose and hypoxia through the expression and secretion of VEGF that are regulated by PKC isozymes. (Young et al., Exp. Eye Res., 80:651-62 (2005).)

To establish the efficacy of PKC-delta peptide inhibitors, similar experiments using rat RPEs are conducted. Peptides inhibitors for delta-PKC are used as described above and show an inhibition of VEGF mRNA production and VEGF secretion when RPEs are exposed to conditions of high glucose and hypoxia.

### Example 2

### Both βKC Isozymes are Required for Angiogenesis

A corneal angiogenesis model was used to demonstrate that both beta PKC isozymes are required for angiogenesis. An ELVAX pellet (DU PONT) containing either VEGF alone (control) or VEGF and a peptide inhibitor of a PKC isozyme (test) was implanted in a rabbit cornea proximal to the limbus capillaries. New blood vessels grow from the limbus toward the implantation site. An angiogenic score was assigned regularly to the test and control corneas. The score is a product of the length and density of the new vessels.

### Scoring the Corneal Angiogenesis Assay

ANGIOGENIC SCORE = vessel density x vessel length.

Vessel density = number of newly formed vessels (score of 0 - 6).

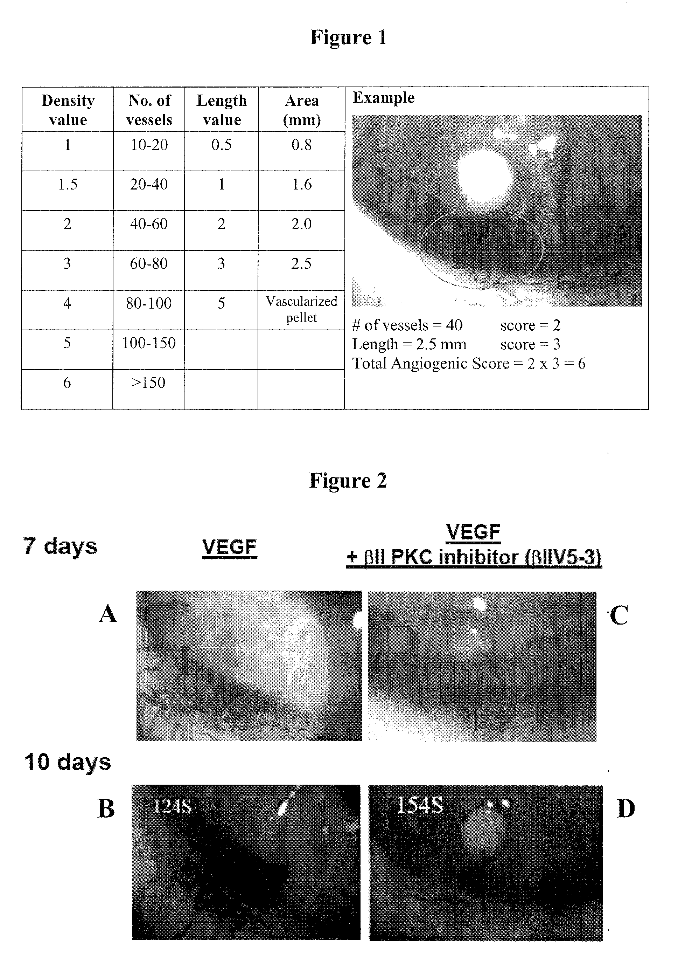

Vessel length = area of neovascularization (mm from the limbus to the pellet) and calculated by a score from 0 - 5. Figure 1 shows an example of the scoring.

In this study, 200 ng of a βPKC inhibitor (β_{I}V5-3, comprising CKLFIMN (SEQ ID NO: 6) conjugated via a disulfide bond to TAT (SEQ ID NO:5), or β_{II}V5-3 comprising CQEVIRN (SEQ ID NO: 7) conjugated via a disulfide bond to TAT (SEQ ID NO:5)) and 200 ng VEGF₁₆₅ were used in the respective pellets.

### β_{II}PKC Specific Inhibitor Prevented New Vessel Growth

Rabbit corneas treated with the β_{II} PKC specific inhibitor substantially prevented neovascularization when measured at days 7 and 10, as shown in Figures 2A-D.

### βPKC Inhibitors Prevented New Vessel Growth

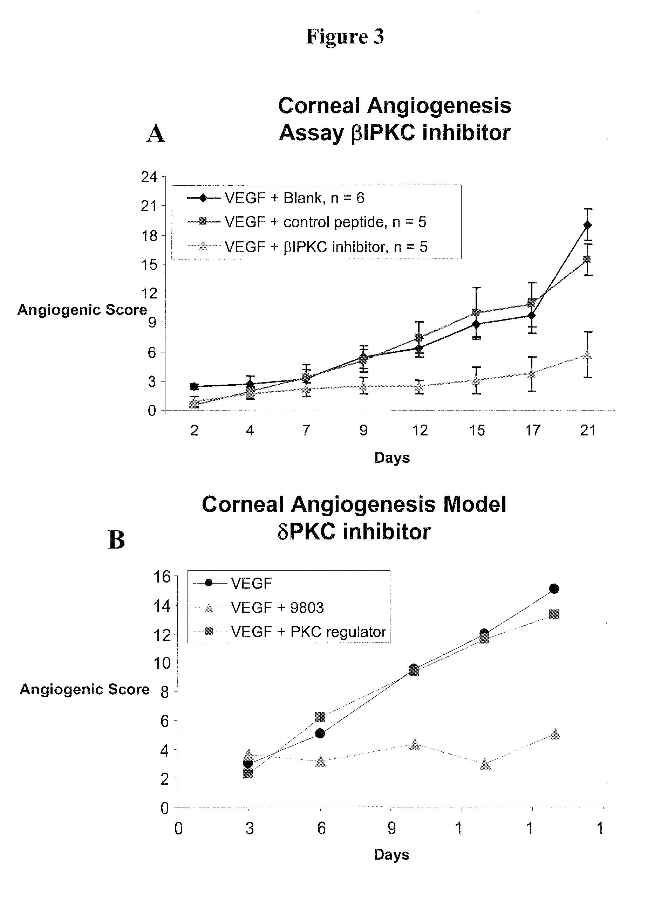

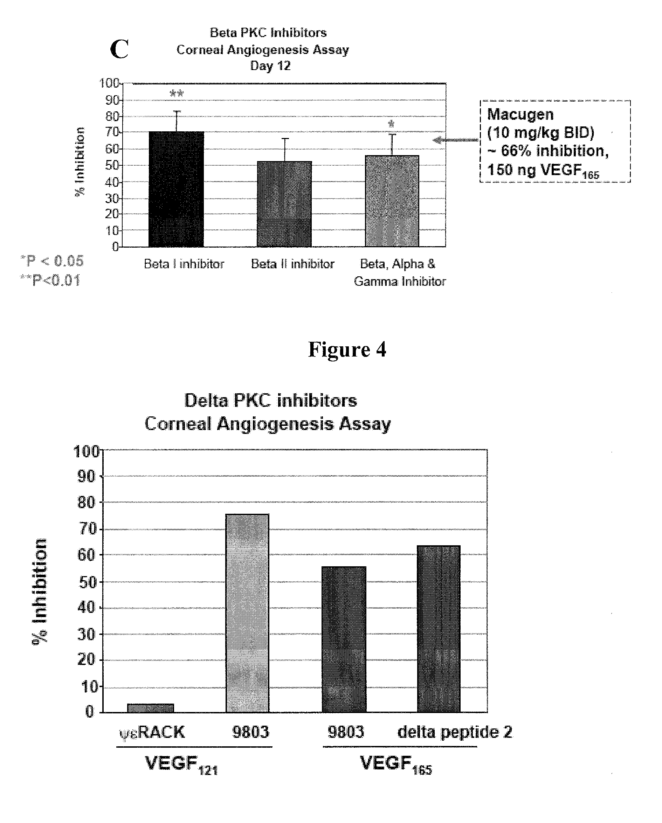

The impact of β_{I} and β_{II} PKC specific inhibitors described above and an alpha, beta, gamma PKC inhibitor βC2-4 comprising CSLNPEWNET (SEQ ID NO: 8) conjugated via a disulfide bond to TAT (SEQ ID NO:5)) was tested in the corneal system. The angiogenic score was measured. The results are shown in Figures 3A-C, where A and B show the scores over time, and C shows the score on day 12. The control peptide for A is a scrambled control 1 peptide having the sequence CPDYHDAGI (SEQ ID NO: 9), while the PKC regulator in B is ΨεRACK, CHDAPIGYD (SEQ ID NO: 10), both conjugated to TAT (SEQ ID NO:5).

The results from these experiments indicate that the activity, at the very least, of both beta PKC isozymes is required for neovascularization.

### Example 3

### Inhibition of delta PKC Reduces VEGF-Induced Angiogenesis

The corneal model described above was used to test the impact of a delta PKC isozyme specific inhibitor on angiogenesis. In this study 200 ng of VEGF (VEGF₁₂₁ or VEGF₁₆₅) in the presence of a control peptide, ΨεRACK, CHDAPIGYD (SEQ ID NO: 10) conjugated to TAT (SEQ ID NO:5), or together with 500 ng of a delta PKC inhibitor, KAI-9803 (comprising CSFNSYELGSL (SEQ ID NO: 11) conjugated via a disulfide bond to TAT (SEQ ID NO:5)) or Peptide 2, dV5, (comprising CYSDKNLIDSM (SEQ ID NO: 12) conjugated via a disulfide bond to TAT (SEQ ID NO:5)), were inserted into the cornea of rabbits. The data from these experiments is shown in Figure 4.

A strong inhibitory response was observed at each time point for two different delta PKC isozyme specific peptides.

### Example 4

### PKC Inhibitors Affect Vascular Permeability

The Miles assay was used to demonstrate that PKC modulators impact VEGF induces vascular permeability. In this study animals were injected intravenously with Evans Blue Dye. Animals were administered VEGF in increasing doses with a PKC modulator intradermally. Plasma extravasation resulted in a visible blue spot on the skin of the subject. The spots were removed, the dye extracted and quantities were measured spectrophotometrically.

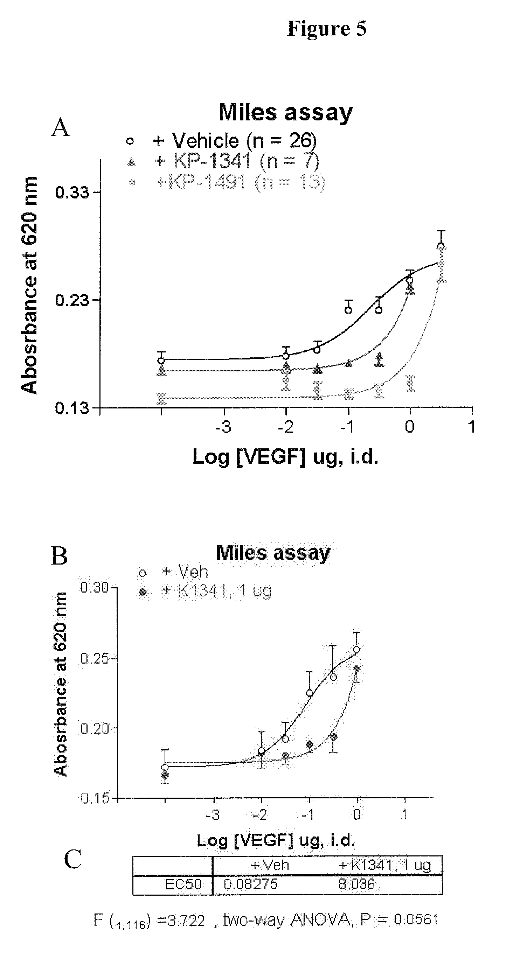

A parent compound, β_{I}V5-3 (comprising CKLFIMN (SEQ ID NO:6) conjugated via a disulfide bond to TAT (SEQ ID NO:5)) and a peptide variant - a linear version of the peptide, linear β_{I}, having the sequence GKLFIMNGGYGRKKRRQRRR (SEQ ID NO: 13) - were tested in the Miles assay. The results are shown in Figures 5A-C, and demonstrate that the assay allowed for the direct comparison of the two β PKC inhibitory peptides and is therefore a useful assay for determining relative potency of peptide modulators in reducing plasma extravasation. Both peptides reduced vascular permeability in comparison to the vehicle alone.

The Miles assay was similarly performed with a β_{I}PKC (β_{I}V5-3 (comprising CKLFIMN (SEQ ID NO:6) conjugated via a disulfide bond to TAT (SEQ ID NO:5)) inhibitor, a β_{II}PKC (β_{II}V5-3 comprising CQEVIRN (SEQ ID NO:7) conjugated via a disulfide bond to TAT (SEQ ID NO:5)) inhibitor, a classical (β-, α-, γPKC) PKC inhibitor (βC2-4 comprising CSLNPEWNET (SEQ ID NO:8) conjugated via a disulfide bond to TAT (SEQ ID NO:5)), and the scrambled control 1 peptide from Example 2 (SEQ ID NO: 9) conjugated to TAT (SEQ ID NO:5).

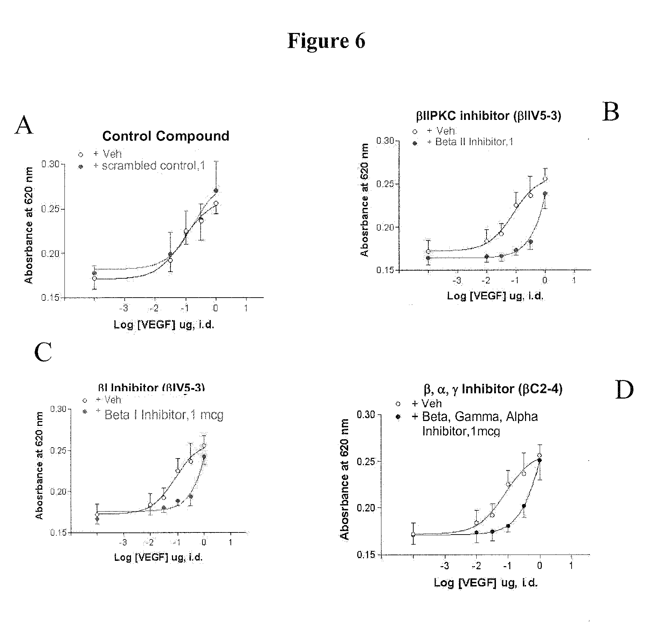

The results of these measurements are shown in Figures 6A-D, and indicate that both β_{I}PKC (β_{I}V5-3 comprising CKLFIMN (SEQ ID NO:6) conjugated via a disulfide bond to TAT (SEQ ID NO:5)) and β_{II}PKC (β_{II}V5-3 comprising CQEVIRN (SEQ ID NO:7) conjugated via a disulfide bond to TAT (SEQ ID NO:5)) inhibitors reduced VEGF-induced permeability in comparison to vehicle alone. Also, the classical PKC inhibitor (βC2-4 comprising CSLNPEWNET (SEQ ID NO:8) conjugated via a disulfide bond to TAT (SEQ ID NO:5)) also reduced VEGF-induced permeability. In contrast, the scrambled control peptide did not reduce VEGF-induced permeability.

### Example 5

### Administration in Rabbits

Radiolabeled TAT peptide [¹⁴C]-YGXaaRKKRRQRRR (SEQ ID NO: 14) (10 µCi) was applied in 5 instillations to each eye (50 µCi total - 4 rabbits, 2 eyes each, n = 8 eyes, 3 mg total applied to each eye). Each application was 1 hour apart, animals sacrificed 15 minutes after last application. Tissue samples were taken and assayed to determine topical distribution. The results are shown below.

### Topical Distribution Data

Vitreous - 365 ng/g tissue (+/- 203) - 0.006% applied total dose.

Choroid - 3.4 µg/g tissue (+/- 3.1) - 0.003% applied total dose.

Retina - 1.6 µg/g tissue (+/- 0.6) - 0.001% applied total dose.

Sclera - 28.4 µ/g tissue (+/- 28.1) - 0.134% applied total dose.

These results show that very little compound reached the posterior segments.

### Example 6

### Administration in Rats

0.8 mg KAI-9706-TAMRA, CHDAPIGYD (SEQ ID NO: 10) conjugated to TAMRA, was delivered via IPV to a rat. 10 minutes post-injection, the animal was euthanized and perfused with formaldehyde. The organs were harvested for immunofluorescent analysis. Red color indicated fluorescence from the TAMRA, blue indicated nuclei (DAPI stain). A number of different sections of the eye were examined for fluorescence, which showed red fluorescence, indicating that the PKC modulatory peptides can be delivered to the eye via IPV injection.

### Example 7

### Intraocular stability

Vitreal fluid was removed from the eye of a recently euthanized pig by inserting a syringe directly into the eye in situ and withdrawing liquid. This fluid was then used for an *"in vitro"* vitreal stability study with KAI-9803 comprising CSFNSYELGSL (SEQ ID NO:11) conjugated via a disulfide bond to TAT (SEQ ID NO:5). A fixed concentration of the peptide was added to 3 tubes containing vitreal fluid and at different times following addition, 5% trichloroacetic acid was added, the tubes spun to remove precipitated material and the supernatant analyzed by HPLC.

Chromatographs were taken from three timepoints. The peak size of the peptide varied, indicating that the stability of the peptides in the eye can be measured and followed *in vitro,* and that this method provides for an *in vitro* assay for determining the vitreal stability of PKC modulatory peptides.

### Example 8

### PKC inhibitory peptide variants

Stability, Miles Assay activity and corneal angiogenesis activities of a number of the peptides were investigated, and scored. The data are shown below:

| **Name** | **Sequence** | **SEQ ID NO:** | **Stability testing** | **Miles activity** | **Corneal activity** |
|---|---|---|---|---|---|
| dV5 | CYGRKKRRQRRR ; CYSDKNLIDSM | SEQ ID NOS: 5, 12 | +/- | ND* | ++ |
| KAI-9803 | CYGRKKRRQRRR ; CSFNSYELGSL | SEQ ID NOS: 5, 11 | + | - | ++ |
| B1 V5-3 | CYGRKKRRQRRR ; CKLFIMN | SEQ ID NOS: 5, 6 | - | ++ | ++ |
| B2 V5-3 | CYGRKKRRQRRR ; CQEVIRN | SEQ ID NOS: 5, 7 | ND | ++ | ++ |
| bC2-4 | CYGRKKRRQRRR ; CSLNPEWNET | SEQ ID NOS: 5, 8 | + | ++ | ++ |
| linear 9803 | GSFNSYELGSLGG YGRKKRRQRRR | SEQ ID NO: 15 | +++ | ND | ND |
| linear bII | GQEVIRNGGYGR KKRRQRRR | SEQ ID NO: 16 | - | + | ND |
| linear bI | GKLFIMNGGYGR KKRRQRRR | SEQ ID NO: 13 | +/- | +++ | ND |
| linear bC2-4 | GSLNPEWNETGG YGRKKRRQRRR | SEQ ID NO: 17 | +++ | ++ | ND |
| bI linear analog | GKLFIMNLSGYGR KKRRQRRR | SEQ ID NO: 18 | ++ | + | ND |
| bII linear analog | GEEVIRNISGYGR KKRRQRRR | SEQ ID NO: 19 | +++ | ND | ND |
| Linear bI (3) | GKLFIMNLDGYG RKKRRQRRR | SEQ ID NO: 20 | ++ | ND | ND |
| Linear bI (4) | GKLFIMNADGYG RKKRRQRRR | SEQ ID NO: 21 | ++ | ND | ND |
| Linear bI (5) | GKLFIMNLGYGR KKRRQRRR | SEQ ID NO: 22 | +++ | +++ | ND |
| Linear bI (6) | GKLFIMNAGGYG RKKRRQRRR | SEQ ID NO: 23 | ++ | ND | ND |

| | | | | | |
|---|---|---|---|---|---|
| * ND = not tested | | | | | |

It will thus be appreciated that the present invention provides the embodiments summarized in the statements below:
1. An isolated protein kinase C (PKC) beta or delta inhibitory peptide, said peptide having activity for the inhibition of angiogenesis and/or the inhibition of vascular permeability.
2. The peptide of statement 1, wherein said peptide has an amino acid sequence comprising between 6 and 15 consecutive residues of SEQ ID NOs: 1, 2 or 3.
3. The peptide of statement 1, wherein said peptide has a sequence selected from the group consisting of SEQ ID NOs: 5 conjugated via a disulfide bond to 6, 5 conjugated via a disulfide bond to 7, 5 conjugated via a disulfide bond to 8, 5 conjugated via a disulfide bond to 11, 5 conjugated via a disulfide bond to 12, and SEQ ID NOs: 12, 13, 15, 16, 17, 18, 19, 20, 21, 22, and 23.
4. The peptide of statement 1, wherein said peptide is conjugated to a carrier.
5. The peptide of statement 4, wherein the peptide has a sequence identified as SEQ ID NO: 6, 7, 8 and 11.
6. The peptide of statement 4, wherein the carrier is selected from the group consisting ofpoly-Arg, TAT, and Drosophila Antennapedia homeodomain.
7. The peptide of statement 1, wherein said peptide comprises an amino acid sequence that has greater than 50% sequence identity with a peptide selected from the group consisting of SEQ ID NOs: 5 conjugated via a disulfide bond to 6, 5 conjugated via a disulfide bond to 7, 5 conjugated via a disulfide bond to 8, 5 conjugated via a disulfide bond to 11, 5 conjugated via a disulfide bond to 12, and SEQ ID NOs:12, 13, 15, 16, 17, 18, 19, 20, 21, 22, and 23.
8. The peptide of statement 1, comprising an isolated linear peptide having greater than 50% sequence identity with a peptide selected from the group consisting of SEQ ID NOs: 13 and 15-23.
9. The peptide of statement 8, wherein said peptide is chemically synthesized.
10. The peptide of statement 8, wherein said peptide is recombinantly produced.
11. The peptide of statement 8, wherein said peptide is selected from the group consisting of SEQ ID Nos:13, and 15-23.
12. An isolated PKC beta I V5 peptide comprising an amino acid sequence that has greater than 50% sequence identity with a peptide selected from the group consisting of SEQ ID Nos: 5 conjugated via a disulphide bond to 6 and SEQ. ID nos: 13, 18, 20, 21, 22 and 23, and having activity as an antagonist of beta I PKC.
13. An isolated PKC beta II V5 peptide comprising an amino acid sequence that has greater than 50% sequence identity with a peptide selected from the group consisting of SEQ ID Nos: 5 conjugated via a disulphide bond to 7 and SEQ. ID nos: 16 and 19, and having activity as an antagonist of beta II PKC.
14. An isolated PKC delta V5 peptide comprising an amino acid sequence that has greater than 50% sequence identity with a peptide selected from the group consisting of SEQ ID Nos 5 conjugated via a disulphide bond to 12 and SEQ. ID No.12, and has activity as an antagonist of delta PKC.
15. A pharmaceutical formulation comprising a pharmaceutically acceptable excipient and a peptide according to any one of statements 1 to 14.
16. An isolated protein kinase C (PKC) inhibitory peptide for use in inhibiting angiogenesis and/or vascular permeability.
17. A peptide according to statement 16, which inhibits a classical PKC isozyme.
18. A peptide according to statement 17, which inhibits beta I PKC.
19. A peptide according to statement 17, which inhibits beta II PKC.
20. A peptide according to statement 16, which is conjugated to a carrier and has greater than 50% sequence identity with a peptide selected from the group consisting of CKLFIMN (SEQ ID NO:6), CQEVIRN (SEQ ID NO:7), and CSLNPEWNET (SEQ ID NO:8).
21. A peptide according to statement 16, which has greater than 50% sequence identity with a peptide selected from a group consisting of SEQ ID Nos. 5 conjugated via a disulfide bond to 6, 5 conjugated via a disulfide bond to 7, 5 conjugated via a disulfide bond to 8 and SEQ ID NOs: 13, 16, 17, 18, 19, 20, 21, 22, and 23.
22. A peptide according to statement 16, which has greater than 50% sequence identity with a peptide selected from a group consisting of SEQ ID Nos: : 5 conjugated via a disulfide bond to 6, 5 conjugated via a disulfide bond to 7, 5 conjugated via a disulfide bond to 8, 5 conjugated via a disulfide bond to 11, 5 conjugated via a disulfide bond to 12, and SEQ ID NOs: 12, 13, 15, 16, 17, 18, 19, 20, 21, 22, and 23.
23. A peptide according to statement 16, which inhibits a novel PKC isozyme.
24. A peptide according to statement 23, which inhibits delta PKC.
25. The peptide according to statement 16, which is CYSDKNLIDSM (SEQ ID NO:12).
26. A peptide according to statement 16, which has greater than 50% sequence identity with CSFNSYELGSL (SEQ ID NO: 11) conjugated to a carrier.
27. A peptide according to statement 16, which is conjugated to a carrier.
28. A peptide according to statement 27, wherein the carrier is selected from poly-Arg, TAT, and the Drosophila Antennapedia homeodomain.
29. A peptide according to statement 16, wherein said use is by administration to an ocular tissue of the subject.
30. A peptide according to statement 29, for use in treating a subject having mascular degeneration.
31. A peptide according to statement 16, wherein said use is by administration to a subject having cancer, diabetic blindness, macular degeneration, rheumatoid arthritis, or psoriasis.
32. A peptide according to statement 16 for use in treating a subject having rheumatoid arthritis.
33. A peptide according to statement 16, wherein said use further comprises use of an anti-angiogenic agent.
34. A peptide according to statement 33, wherein the anti-angiogenic agent inhibits at least one of the group consisting of VEGF, FGF, PDGFB, EGF, LPA, HGF, PD-ECT, IL-8, angiogenin, TNF-alpha, TGF-beta, TGF-alpha, proliferin and PLGF.

## Claims

1. An isolated delta protein kinase C (PKC) inhibitory peptide for use in inhibiting angiogenesis in a subject in need thereof, wherein said inhibitory peptide has an amino acid sequence that is at least 90-95% identical to SEQ ID NO:11 1 or SEQ ID NO:15.

2. The peptide of claim 1 which has the amino acid sequence of SEQ ID NO:11

3. The peptide of claim 1 which has the amino acid sequence of SEQ ID NO:15.

4. A peptide as claimed in claim 1 or 2 which is conjugated to a carrier via a disulfide bond.

5. A peptide as claimed in claim 4, wherein the carrier is a poly-Arg peptide, a TAT peptide, or a Drosophila Antennapedia homeodomain peptide.

6. A peptide as claimed in claim 4, wherein an isolated delta PKC inhibitory comprising a peptide of SEQ ID NO:11 is conjugated via a disulfide bond to a carrier peptide.

7. A peptide as claimed in claim 6, wherein the carrier peptide comprises the amino acid sequence of SEQ ID NO:5.

8. The peptide of claim 1, wherein the isolated delta PKC inhibitory peptide consists of a first peptide consisting of the amino acid sequence of SEQ ID NO:11 and a second peptide consisting of the amino acid sequence of SEQ ID NO:5, wherein the first and second peptides are cross-linked via a disulfide bond.

9. A peptide according to any one of claims 1-8, wherein said use comprises contacting said peptide with a cell which is an angiogenic endothelial cell.

10. A peptide according to any one of the preceding claims, wherein said use comprises use of said peptide together with an anti-angiogenic agent.

11. A peptide according to claim 11, wherein the anti-angiogenic agent inhibits at least one of VEGF, FGF, PDGFB, EGF, LPA, HGF, PD-ECF, IL-8, angiogenin, TNF-alpha, TGF-beta, TGF-alpha, proliferin and PLGF.

12. A composition comprising an isolated delta protein kinase C (PKC) inhibitory peptide and a pharmaceutically acceptable excipient, wherein said inhibitory peptide has an amino acid sequence that is at least 90-95% identical to SEQ ID NO:11 or SEQ ID NO:15.

13. A composition as claimed in claim 12, wherein said inhibitory peptide has the amino acid sequence of SEQ ID NO:11.

14. A composition as claimed in claim 12, wherein said inhibitory peptide has the amino acid sequence of SEQ ID NO:15.

15. A composition as claimed in claim 12 or 13, wherein said peptide is conjugated to a carrier via a disulfide bond.

16. A composition as claimed in claim 15, wherein the carrier is a poly-Arg peptide, a TAT peptide, or a Drosophila Antennapedia homeodomain peptide.

17. A composition of claim 15, wherein an isolated delta PKC inhibitory peptide comprising a peptide of SEQ ID NO:11 is conjugated via a disulfide bond to a carrier peptide.

18. A composition as claimed in 17, wherein the carrier peptide comprises the amino acid sequence of SEQ ID NO:5.

19. A composition as claimed in claim 12, wherein the isolated delta PKC inhibitory peptide consists of a first peptide consisting of the amino acid sequence of SEQ ID NO:11 and a second peptide consisting of the amino acid sequence of SEQ ID NO:5, wherein the first and second peptides are cross-linked via a disulfide bond.

20. A composition according to any one of claims 12 to 19 for use in inhibiting angiogenesis in a subject in need thereof.

21. A composition as claimed in claim 20 which further comprises an anti-angiogenic agent.

22. A composition as claimed in claim 21, wherein the anti-angiogenic agent inhibits at least one of VEGF, FGF, PDGFB, EGF, LPA, HGF, PD-ECF, IL-8, angiogenin, TNF-alpha, TGF-beta, TGF-alpha, proliferin and PLGF.


SEQUENCE LISTING

<110> KAI PHARMACEUTICALS, INC.


<120> PROTEIN KINASE C PEPTIDE MODULATORS OF ANGIOGENESIS

<130> CI/PX205185EPA

<140> Divisional of EPA 06814988.9
<141> 2006-09-19

<150> US 60/718,508
<151> 2005-09-19

<160> 23

<170> FastSEQ for Windows Version 4.0

<210> 1
<211> 54
<212> PRT
<213> Artificial Sequence

<220>
<223> Synthetic construct

<220>
<221> PEPTIDE
<222> (1)...(54)
<223> beta I PKC V5 domain

<400> 1
Lys Pro Lys Ala Arg Asp Lys Arg Asp Thr Ser Asn Phe Asp Lys Glu
 1               5                   10                  15
Phe Thr Arg Gln Pro Val Glu Leu Thr Pro Thr Asp Lys Leu Phe Ile
            20                  25                  30
Met Asn Leu Asp Gln Asn Glu Phe Ala Gly Phe Ser Tyr Thr Asn Pro
        35                  40                  45
Glu Phe Val Ile Asn Val
    50


<210> 2
<211> 56
<212> PRT
<213> Artificial Sequence

<220>
<223> Synthetic construct

<220>
<221> PEPTIDE
<222> (1)...(56)
<223> beta II PKC V5 domain

<400> 2
Lys Pro Lys Ala Cys Gly Arg Asn Ala Glu Asn Phe Asp Arg Phe Phe
 1               5                   10                  15
Thr Arg His Pro Pro Val Leu Thr Pro Pro Asp Gln Glu Val Ile Arg
            20                  25                  30
Asn Ile Asp Gln Ser Glu Phe Glu Gly Phe Ser Phe Val Asn Ser Glu
        35                  40                  45
Phe Leu Lys Pro Glu Val Lys Ser
    50                  55


<210> 3

<211> 56
<212> PRT
<213> Artificial Sequence

<220>
<223> Synthetic construct

<220>
<221> PEPTIDE
<222> (1)...(56)
<223> delta PKC V5 domain

<400> 3
Arg Pro Lys Val Lys Ser Pro Arg Asp Tyr Ser Asn Phe Asp Gln Glu
 1               5                  10                  15
Phe Leu Asn Glu Lys Ala Arg Leu Ser Tyr Ser Asp Lys Asn Leu Ile
            20                  25                  30
Asp Ser Met Asp Gln Ser Ala Phe Ala Gly Phe Ser Phe Val Asn Pro
        35                  40                  45
Lys Phe Glu His Leu Leu Glu Asp
    50                  55


<210> 4
<211> 11
<212> PRT
<213> Artificial Sequence

<220>
<223> Synthetic construct

<220>
<221> PEPTIDE
<222> (1)...(11)
<223> TAT carrier

<400> 4
Tyr Gly Arg Lys Lys Arg Arg Gln Arg Arg Arg
 1               5                  10


<210> 5
<211> 12
<212> PRT
<213> Artificial Sequence

<220>
<223> Synthetic construct

<220>
<221> PEPTIDE
<222> (1)...(12)
<223> TAT carrier with N-terminal cysteine

<400> 5
Cys Tyr Gly Arg Lys Lys Arg Arg Gln Arg Arg Arg
 1               5                  10


<210> 6
<211> 7
<212> PRT
<213> Artificial Sequence

<220>
<223> Synthetic construct

<220>
<221> PEPTIDE

<222> (1)...(7)
<223> PKC inhibitor

<400> 6
Cys Lys Leu Phe Ile Met Asn
 1               5


<210> 7
<211> 7
<212> PRT
<213> Artificial Sequence

<220>
<223> Synthetic construct

<220>
<221> PEPTIDE
<222> (1)...(9)
<223> PKC inhibitor

<400> 7
Cys Gln Glu Val Ile Arg Asn
 1               5


<210> 8
<211> 10
<212> PRT
<213> Artificial Sequence

<220>
<223> Synthetic construct

<220>
<221> PEPTIDE
<222> (1)...(10)
<223> PKC inhibitor

<400> 8
Cys Ser Leu Asn Pro Glu Trp Asn Glu Thr
 1               5                   10


<210> 9
<211> 9
<212> PRT
<213> Artificial Sequence

<220>
<223> Synthetic construct

<220>
<221> PEPTIDE
<222> (1)...(9)
<223> scrambled control 1 peptide

<400> 9
Cys Pro Asp Tyr His Asp Ala Gly Ile
 1               5


<210> 10
<211> 9
<212> PRT
<213> Artificial Sequence

<220>
<223> Synthetic construct

<220>
<221> PEPTIDE
<222> (1)...(9)
<223> PKC regulator

<400> 10
Cys His Asp Ala Pro Ile Gly Tyr Asp
 1               5


<210> 11
<211> 11
<212> PRT
<213> Artificial Sequence

<220>
<223> Synthetic construct

<220>
<221> PEPTIDE
<222> (1)...(11)
<223> PKC inhibitor

<400> 11
Cys Ser Phe Asn Ser Tyr Glu Leu Gly Ser Leu
 1               5                   10


<210> 12
<211> 11
<212> PRT
<213> Artificial Sequence

<220>
<223> Synthetic construct

<220>
<221> PEPTIDE
<222> (1)...(11)
<223> PKC inhibitor

<400> 12
Cys Tyr Ser Asp Lys Asn Leu Ile Asp Ser Met
 1               5                   10


<210> 13
<211> 20
<212> PRT
<213> Artificial Sequence

<220>
<223> Synthetic construct

<220>
<221> PEPTIDE
<222> (1)...(20)
<223> linear beta I

<400> 13
Gly Lys Leu Phe Ile Met Asn Gly Gly Tyr Gly Arg Lys Lys Arg Arg
 1               5                   10                  15
Gln Arg Arg Arg
            20


<210> 14
<211> 14

<212> PRT
<213> Artificial Sequence

<220>
<223> Synthetic construct

<220>
<221> PEPTIDE
<222> (1)...(14)
<223> radiolabeled TAT peptide

<220>
<221> SITE
<222> 1
<223> carbon 14 radiolabel modification

<220>
<221> VARIANT
<222> 3
<223> Xaa = any natural amino acid

<400> 14
Tyr Gly Xaa Ala Ala Arg Lys Lys Arg Arg Gln Arg Arg Arg
 1               5                   10


<210> 15
<211> 24
<212> PRT
<213> Artificial Sequence

<220>
<223> Synthetic construct

<220>
<221> PEPTIDE
<222> (1)...(24)
<223> PKC inhibitor linear 9803

<400> 15
Gly Ser Phe Asn Ser Tyr Glu Leu Gly Ser Leu Gly Gly Tyr Gly Arg
 1               5                   10                  15
Lys Lys Arg Arg Gln Arg Arg Arg
            20


<210> 16
<211> 20
<212> PRT
<213> Artificial Sequence

<220>
<223> Synthetic construct

<220>
<221> PEPTIDE
<222> (1)...(20)
<223> PKC inhibitor linear beta II

<400> 16
Gly Gln Glu Val Ile Arg Asn Gly Gly Tyr Gly Arg Lys Lys Arg Arg
 1               5                   10                  15
Gln Arg Arg Arg
            20


<210> 17
<211> 23
<212> PRT

<213> Artificial Sequence

<220>
<223> Synthetic construct

<220>
<221> PEPTIDE
<222> (1)...(23)
<223> PKC inhibitor linear beta C2-4

<400> 17
Gly Ser Leu Asn Pro Glu Trp Asn Glu Thr Gly Gly Tyr Gly Arg Lys
 1               5                   10                  15
Lys Arg Arg Gln Arg Arg Arg
            20


<210> 18
<211> 21
<212> PRT
<213> Artificial Sequence

<220>
<223> Synthetic construct

<220>
<221> PEPTIDE
<222> (1)...(21)
<223> PKC inhibitor beta I linear analog

<400> 18
Gly Lys Leu Phe Ile Met Asn Leu Ser Gly Tyr Gly Arg Lys Lys Arg
 1               5                   10                  15
Arg Gln Arg Arg Arg
            20


<210> 19
<211> 21
<212> PRT
<213> Artificial Sequence

<220>
<223> Synthetic construct

<220>
<221> PEPTIDE
<222> (1)...(21)
<223> PKC inhibitor beta II linear analog

<400> 19
Gly Glu Glu Val Ile Arg Asn Ile Ser Gly Tyr Gly Arg Lys Lys Arg
 1               5                   10                  15
Arg Gln Arg Arg Arg
            20


<210> 20
<211> 21
<212> PRT
<213> Artificial Sequence

<220>
<223> Synthetic construct

<220>
<221> PEPTIDE
<222> (1)...(22)
<223> PKC inhibitor linear beta I (3)

<400> 20
Gly Lys Leu Phe Ile Met Asn Leu Asp Gly Tyr Gly Arg Lys Lys Arg
1               5                   10                  15
Arg Gln Arg Arg Arg
            20


<210> 21
<211> 21
<212> PRT
<213> Artificial Sequence

<220>
<223> Synthetic construct

<220>
<221> PEPTIDE
<222> (1)...(21)
<223> PKC inhibitor linear beta I (4)

<400> 21
Gly Lys Leu Phe Ile Met Asn Ala Asp Gly Tyr Gly Arg Lys Lys Arg
1               5                   10                  15
Arg Gln Arg Arg Arg
            20


<210> 22
<211> 20
<212> PRT
<213> Artificial Sequence

<220>
<223> Synthetic construct

<220>
<221> PEPTIDE
<222> (1)...(20)
<223> PKC inhibitor linear beta I (5)

<400> 22
Gly Lys Leu Phe Ile Met Asn Leu Gly Tyr Gly Arg Lys Lys Arg Arg
1               5                   10                  15
Gln Arg Arg Arg
            20


<210> 23
<211> 21
<212> PRT
<213> Artificial Sequence

<220>
<223> Synthetic construct

<220>
<221> PEPTIDE
<222> (1)...(21)
<223> PKC inhibitor linear beta I (6)

<400> 23
Gly Lys Leu Phe Ile Met Asn Ala Gly Gly Tyr Gly Arg Lys Lys Arg
1               5                   10                  15
Arg Gln Arg Arg Arg
            20